# FASCICULE DE BREVET EUROPEEN

(11) **EP 1 981 835 B1**
(45) Date de publication et mention de la délivrance du brevet: **06.05.2009**
(21) Numéro de dépôt: 07731584.4
(22) Date de dépôt: 06.02.2007
(51) Int. Cl.: C07C 57/055, C07C 45/52, C07C 45/35, C07C 57/05

(54) **PROCEDE DE PREPARATION D'ACIDE ACRYLIQUE**
HERSTELLUNGSVERFAHREN FÜR ACRYLSÄURE
ACRYLIC ACID PREPARATION METHOD

(30) Priorité: 07.02.2006 FR 0601061
(43) Date de publication de la demande: 22.10.2008
(73) Titulaire: Arkema France, 92700 Colombes (FR)
(72) Inventeur: DUBOIS, Jean-Luc, F-69390 Millery (FR)
(74) Mandataire: Bonnel, Claudine
(86) Numéro de dépôt international: PCT/FR2007/050758
(87) Numéro de publication internationale: WO 2007/090991

(56) Documents cités:
- EP-A1- 0 257 565
- WO-A-20/04099113
- DE-C1- 4 238 493
- GB-A- 1 152 215
- DATABASE WPI Week 200560 Derwent Publications Ltd., London, GB; AN 2005-585659 XP002405550 & WO 2005/073160 A1 (NIPPON SHOKUBAI CO LTD) 11 août 2005 (2005-08-11) cité dans la demande

## Description

### Domaine technique

La présente invention concerne un procédé de préparation d'acide acrylique à partir du propylène comprenant une première étape d'oxydation du propylène en acroléine et une seconde étape d'oxydatio n de l'acroléine en acide acrylique, dans lequel on met en oeuvre une étape de déshydratation de glycérol en présence d'un gaz contenant du propylène, et plus particulièrement en présence du gaz de réaction issu de l'étape d'oxydation du propylène en acroléine.

### Art antérieur et problème technique

Le procédé de synthèse classique de l'acide acrylique utilise une réaction catalytique du propylène à l'aide d'un mélange contenant de l'oxygène. Cette réaction est conduite généralement en phase vapeur, et le plus souvent en deux étapes :
- La première étape réalise l'oxydation sensiblement quantitative du propylène en un mélange riche en acroléine, dans lequel l'acide acrylique est minoritaire,
- La deuxième étape réalise l'oxydation sélective de l'acroléine en acide acrylique.
Les conditions de réaction de ces deux étapes, réalisées dans deux réacteurs en série, sont différentes et nécessitent des catalyseurs adaptés à la réaction. Il n'est pas nécessaire d'isoler l'acroléine au cours de ce procédé en deux étapes. La réaction peut également être conduite dans un seul réacteur, mais, dans ce cas, il est nécessaire de séparer et recycler de grandes quantités d'acroléine à l'étape d'oxydation.

Dans un certain nombre de cas, il peut être intéressant de pouvoir augmenter les capacités de production en acide acrylique des unités existantes.

La production d'acide acrylique est fortement dépendante de la matière première propylène. Le propylène, obtenu par vapocraquage ou craquage catalytique de coupes pétrolières a l'inconvé nient de contribuer à l'augmentation de l'effet de serre, du fait de son origine fossile. Par ailleurs, la ressource en propylène peut devenir limitée. Il apparaît donc particulièrement intéressant de pouvoir augmenter la productivité en acide acrylique, tout en réduisant la dépendance à une ressource fossile.

Il est connu depuis longtemps que le glycérol peut conduire à l'obtention d'acroléine. Le glycérol est issu de la méthanolyse des huiles végétales en même temps que les esters méthyliques qui sont, eux, employés notamment comme carburants ou combustibles dans le gazole et le fioul domestique. C'est un produit naturel qui jouit d'une aura "verte", il est disponible en grande quantité et peut être stocké et transporté sans difficulté. De nombreuses études sont consacrées à la valorisation du glycérol selon son degré de pureté, et la déshydratation du glycérol en acroléine est une des voies envisagées.

La réaction mise en jeu pour obtenir l'acroléine à partir du glycérol est :

CH₂OH-CHOH-CH₂OH ⇔ CH₂=CH-CHO + 2H₂O

En règle générale la réaction d'hydratation est favorisée aux basses températures, et la réaction de déshydratation est favorisée aux températures élevées. Pour obtenir l'acroléine, il faut donc mettre en oeuvre une température suffisante, et/ou un vide partiel pour déplacer la réaction. La réaction peut être effectuée en phase liquide ou en phase gaz. Ce type de réaction est connu pour être catalysée par des acides. Différents procédés de synthèse de l'acroléine à partir du glycérol sont décrits dans l'art antérieur ; on peut citer notamment les documents FR 695931, US 2,558,520, WO 99/05085, US 5,387,720.

Le document WO 2005/073160 décrit un procédé de préparation d'acide acrylique à partir de glycérol en deux étapes, la première étape consistant à soumettre le glycérol à une réaction de déshydratation en phase gaz, la seconde étape consistant à soumettre le produit de réaction gazeux ainsi obtenu à une réaction d'oxydation en phase gaz. Le document EP-A-0257565 décrit un procédé de préparation d'acide acrylique à partir de propylène en deux étapes.

Il a maintenant été trouvé que la réaction de déshydratation du glycérol en acroléine peut s'effectuer en présence d'un gaz contenant du propylène, et plus particulièrement en présence du gaz de réaction issu de l'étape d'oxydation du propylène en acroléine. Il est ainsi avantageux d'introduire du glycérol dans le procédé conventionnel d'oxydation catalytique en phase gazeuse du propylène en deux étapes pour préparer de l'acide acrylique, ce qui permet d'utiliser une matière première renouvelable tout en augmentant la production d'acide acrylique. Un tel procédé répond alors aux critères associés au nouveau concept de "chimie verte" dans un cadre plus global de développement durable.

### Exposé de l'invention

L'objet de la présente invention est donc un procédé de préparation d'acide acrylique à partir du propylène comprenant une première étape d'oxydation du propylène en acroléine et une seconde étape d'oxydation de l'acroléine en acide acrylique, **caractérisé en ce qu**'il comprend une étape de déshydratation de glycérol en présence d'un gaz contenant du propylène.

De préférence, le gaz contenant du propylène est le gaz de réaction issu de l'étape d'oxydation du propylène en acroléine.

Sans que la demanderesse soit tenue à une quelconque explication, elle pense que l'étape de déshydratation du glycérol permet de refroidir les gaz de réaction issus de la première étape d'oxydation du propylène en acroléine, avant d'effectuer la seconde étape d'oxydation de l'acroléine en acide acrylique.

En effet, dans la réaction d'oxydation du propylène en acroléine, les gaz de réaction sortent de la zone de réaction à une température élevée, la réaction d'oxydation du propylène étant exothermique. Dans un procédé de préparation d'acide acrylique à partir du propylène en deux étapes, il est nécessaire de refroidir les gaz de réaction issus de la première étape d'oxydation du propylène en acroléine avant d'entrer dans le second étage d'oxydation de l'acroléine en acide acrylique, car la réaction d'oxydation de l'acroléine en acide acrylique s'effectue à une température plus basse que la réaction d'oxydation du propylène en acroléine. De plus, l'acroléine peut s'auto-enflammer aux températures élevées entraînant des pertes de rendements.

Ce refroidissement est en général obtenu grâce à un échangeur thermique placé en aval de la zone catalytique du premier étage. Le même effet peut, en tout ou partie, être obtenu grâce à la mise en oeuvre d'une réaction endothermique, telle que la déshydratation du glycérol. Dans la présente invention, la réaction de déshydratation du glycérol présente l'avantage de conduire au même produit de réaction principal (acroléine) que la réaction d'oxydation du propylène. Il en résulte ainsi une augmentation de la productivité en acroléine tout en récupérant de manière efficace la chaleur de la réaction d'oxydation, et par conséquent une augmentation de la productivité en acide acrylique.

D'autres caractéristiques et avantages de l'invention ressortiront mieux à la lecture de la description qui suit et en référence aux figures annexées dans lesquelles :
- Les Figures 1, 2 et 3 représentent schématiquement les configurations conventionnelles pour l'oxydation du propylène en acide acrylique en deux étapes.
- Les Figures 4 et 5 représentent schématiquement différentes configurations correspondant à des modes de réalisation du procédé selon l'invention.

### Exposé détaillé de l'invention

Dans le procédé de l'invention, l'étape de déshydratation de glycérol est effectuée en phase gaz en présence d'un catalyseur à une température allant de 150°C à 500°C, de préférence comprise entre 250°C et 350°C, et une pression comprise entre 1 et 5 bars.

L'étape de déshydratation de glycérol s'effectue en amont de la réaction d'oxydation catalytique du propylène en acroléine en présence du gaz d'alimentation comprenant le propylène, ou en aval de la réaction d'oxydation catalytique du propylène en acroléine, en présence du mélange gazeux issu de cette réaction. Elle peut être intégrée directement dans le réacteur d'oxydation ou être réalisée dans un réacteur placé immédiatement en amont ou en aval du réacteur d'oxydation du propylène en acroléine. La réaction de déshydratation étant faiblement endothermique, il n'est pas nécessaire d'avoir un lit multitubulaire pour cette réaction. Le lit fixe conventionnel ainsi qu'une configuration en modules (plaques ou paniers) peuvent convenir. Les modules présentent l'avantage de pouvoir être chargés et déchargés facilement lorsque le catalyseur est désactivé.

Les catalyseurs qui conviennent sont des matériaux homogènes ou multiphases, insolubles dans le milieu réactionnel qui ont une acidité de Hammett, notée H₀ inférieure à +2. Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse. Les catalyseurs répondant au critère d'acidité H₀ inférieur à +2, peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides.

Avantageusement, les catalyseurs sont choisis parmi les zéolithes, les composites Nafion^{®} (à base d'acide sulfonique de polymères fluorés), les alumines chlorées, les acides et sels d'acides phosphotungstiques et/ou silicotungstiques, et différents solides de type oxydes métalliques tels que oxyde de tantale Ta₂O₅, oxyde de niobium Nb₂O₅, alumine Al₂O₃, oxyde de titane TiO₂, zircone ZrO₂, oxyde d'étain SnO₂, silice SiO₂ ou silico-aluminate SiO₂-Al₂O₃, imprégnés de fonctions acides telles que borate BO₃, sulfate SO₄, tungstate WO₃, phosphate PO₄, silicate SiO₂, ou molybdate MoO₃. Selon les données de la littérature, ces catalyseurs ont tous une acidité de Hammett H₀ inférieure à +2.

Les catalyseurs préférés sont les zircones sulfatées, les zircones phosphatées, les zircones tungstées, les zircones silicées, les oxydes de titane ou d'étain sulfatés, les alumines ou silices phosphatées.

Ces catalyseurs ont tous une acidité de Hammett H₀ inférieure à +2, l'acidité H₀ peut alors varier dans une large mesure, jusqu'à des valeurs pouvant atteindre -20 dans l'échelle de référence avec les indicateurs de Hammett. Le tableau donné à la page 71 de la publication sur la catalyse acido-basique (C. Marcilly) Vol 1 aux Editions Technip (n°ISBN 2-7108-0841-2) illustre des exemples de catalyseurs solides dans cette gamme d'acidité.

Le glycérol est utilisé pur, ou sous forme de solution aqueuse concentrée ou diluée. Avantageusement, on utilise du glycérol pur ou une solution aqueuse de glycérol de concentration allant de 10% à 100% en poids, de préférence allant de 50% à 100% lorsque utilisé en aval de l'oxydation du propylène ; la vapeur d'eau présente dans le gaz de réaction issu de l'étape d'oxydation du propylène en acroléine permet de diluer la solution de glycérol et d'éviter ainsi des réactions parasites comme la formation d'éthers de glycérol ou des réactions entre l'acroléine produite et le glycérol. Dans le mode de réalisation de l'invention où l'étape de déshydratation de glycérol s'effectue en amont de la réaction d'oxydation catalytique du propylène, on peut utiliser une solution aqueuse de glycérol, de préférence de concentration allant de 10% à 50% en poids, plus particulièrement de 15% à 30% en poids.

Le glycérol peut être injecté sous forme liquide ou sous forme gazeuse. L'injection sous forme liquide permet de bénéficier de la chaleur latente de vaporisation du glycérol, permettant ainsi de refroidir les gaz issus de l'étape en amont d'oxydation du propylène. Dans ce cas, le catalyseur de déshydratation peut être précédé d'un lit d'inertes sur lequel s'effectue la vaporisation du glycérol. Il peut être injecté sous forme gazeuse à une température inférieure à celle des gaz sortant de la zone d'oxydation, ce qui permet d'accentuer encore l'effet de refroidissement de ces gaz. Par ailleurs, le glycérol peut être injecté sous pression de sorte que la détente du gaz permet une consommation supplémentaire de chaleur.

Le mélange gazeux qui alimente le réacteur pour la première étape d'oxydation du propylène en acroléine est généralement constitué de propylène, de vapeur d'eau, d'un gaz inerte pouvant être de l'azote ou de l'argon, et d'oxygène moléculaire ou d'un gaz contenant de l'oxygène moléculaire.

Le gaz de réaction issu de l'étape d'oxydation du propylène en acroléine est généralement constitué d'un mélange des gaz n'ayant pas réagi (propylène non transformé, propane présent initialement dans le propylène, gaz inerte, vapeur d'eau, oxygène, CO, CO₂), d'acroléine produite et de différents sous-produits tels que acide acrylique, acide acétique et autres composés minoritaires.

La réaction de déshydratation du glycérol dans le procédé selon l'invention s'effectue par conséquent en présence d'oxygène moléculaire qui se trouve, soit dans le mélange gazeux qui alimente le réacteur pour la première étape d'oxydation du propylène en acroléine, soit dans le mélange gazeux issu de l'étape d'oxydation du propylène en acroléine. L'oxygène moléculaire peut être présent sous forme d'air ou sous forme d'un mélange de gaz contenant de l'oxygène moléculaire. Selon un mode de réalisation de l'invention, il est possible d'ajouter une quantité supplémentaire d'oxygène moléculaire ou d'un gaz contenant de l'oxygène moléculaire pour l'étape de déshydratation du glycérol. La quantité d'oxygène est choisie de façon à être en dehors du domaine d'inflammabilité en tout point de l'installation. La présence d'oxygène permet de limiter la désactivation du catalyseur de déshydratation par cokage. De plus, l'ajout d'oxygène améliore le rendement de la réaction pour de nombreux systèmes catalytiques.

La réaction d'oxydation catalytique du propylène en acide acrylique en deux étapes dans le procédé selon l'invention s'effectue selon des conditions connues de l'homme du métier, en faisant passer un mélange gazeux pouvant comporter du propylène, de la vapeur d'eau, un gaz inerte pouvant être de l'azote ou de l'argon et de l'oxygène moléculaire ou un gaz contenant de l'oxygène moléculaire, sur un catalyseur d'oxydation du propylène pour obtenir un mélange gazeux riche en acroléine. Puis, la réaction d'oxydation sélective de l'acroléine en acide acrylique s'effectue sur un catalyseur adapté d'oxydation de l'acroléine. Le procédé peut être mis en oeuvre dans un seul réacteur ou dans deux réacteurs placés en série. Les réacteurs sont généralement des réacteurs multitubulaires en lit fixe. Il est possible aussi d'utiliser un échangeur à plaques avec un agencement modulaire du catalyseur tel que décrit dans les documents EP 995491, EP 1147807 ou US 2005/0020851.

Dans le cas où l'oxydation catalytique du propylène s'effectue en présence d'un ballast thermique, telle que décrite par exemple dans le document EP 293 224 A1, permettant la mise en oeuvre d'un débit de propylène plus élevé, le mélange gazeux issu de la réaction possède une chaleur spécifique Cp plus élevée. Le procédé sebn l'invention est particulièrement avantageux dans ce cas pour évacuer l'excès de chaleur transporté par les gaz de réaction.

Un mode de réalisation préféré de l'invention consiste à utiliser du propane comme gaz inerte en substitution de tout ou partie de l'azote de l'air. Le propane, grâce à sa chaleur spécifique plus élevée amène plus de calories vers le réacteur, ce qui permet d'effectuer la réaction de déshydratation du glycérol plus facilement. Le gaz issu de l'étape de déshydratation contient alors comme constituants principaux de la vapeur d'eau, du propane, de l'acroléine et de l'oxygène résiduel. Ce gaz alimente alors directement l'étage d'oxydation de l'acroléine en acide acrylique. Dans ce cas, le propane est utile pour emmener des calories de la réaction d'oxydation qui est fortement exothermique. Après absorption de l'acide acrylique, les gaz riches en propane peuvent être recyclés vers l'étape de déshydratation. De préférence, le gaz subit des traitements de purification afin d'éliminer des impuretés qui peuvent être gênantes pour les réactions de déshydratation et d'oxydation, telles que le CO et/ou CO₂, et afin de limiter la concentration de ces impuretés dans la boucle de recycle. Dans ce cas, il est particulièrement avantageux de maîtriser la concentration en argon dans la boucle de gaz du fait de sa très faible chaleur spécifique. Comme techniques de séparation utilisables seules ou en combinaison, on peut citer l'oxydation sélective du CO en CO₂, le lavage aux amines, le lavage à la potasse, les techniques d'adsorption, la séparation membranaire ou la séparation cryogénique.

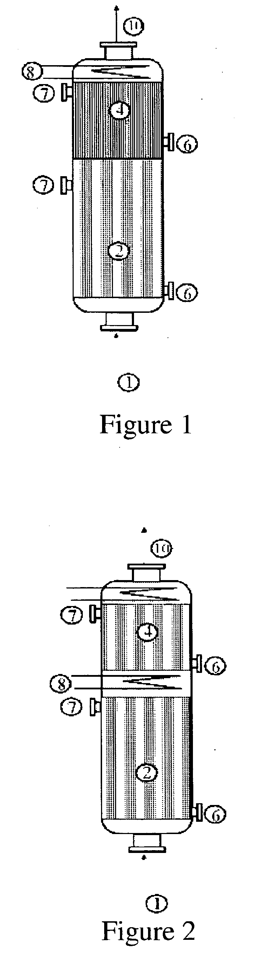

En référence aux Figures 1 et 2, dans un procédé conventionnel d'oxydation du propylène en acide acrylique en deux étapes dans un seul réacteur, on fait passer dans un réacteur multitubulaire du bas vers le haut, un mélange gazeux 1 comportant du propylène, de la vapeur d'eau, de l'azote et de l'oxygène moléculaire, sur un catalyseur 2 d'oxydation du propylène. Le mélange issu de cette réaction, comprenant les gaz n'ayant pas réagi, l'acroléine produite et des sous-produits, passe ensuite sur un catalyseur 4 d'oxydation de l'acroléine en acide acrylique. On obtient un mélange 10 comprenant l'acide acrylique produit, de l'acroléine résiduelle, les gaz n'ayant pas réagi, de l'eau et des sous-produits. Des fluides réfrigérants circulent en 6 et 7 de façon à maintenir une température de réaction pouvant être comprise entre 300°C et 380°C pour la réaction d'oxydation du propylène en acroléine et une température pouvant être comprise entre 260°C et 320°C pour l'oxydation de l'acroléine en acide acrylique. Un échangeur thermique 8 permettant de refroidir les gaz de réaction est placé en aval des deux étapes d'oxydation, comme sur la Figure 1 ; de préférence, l'échangeur thermique 8 de la figure 1 est en aval du réacteur et non dans le réacteur, ce qui facilite le chargement et déchargement du réacteur. Un second échangeur thermique 8 peut être placé entre les deux lits catalytiques, comme représenté sur la Figure 2, permettant de refroidir le mélange gazeux intermédiaire.

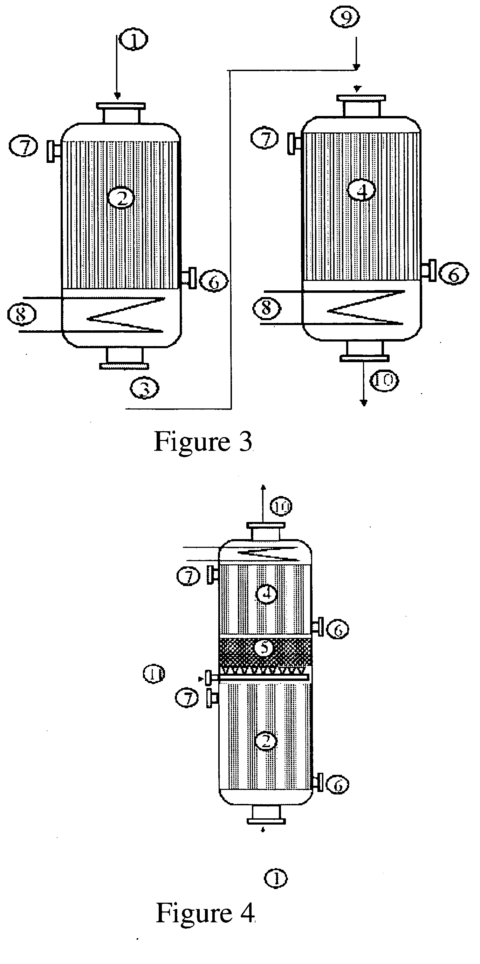

En référence à la Figure 3, dans un procédé conventionnel d'oxydation du propylène en acide acrylique en deux étapes dans deux réacteurs consécutifs, on fait passer dans un premier réacteur multitubulaire du haut vers le bas, un mélange gazeux 1 comportant du propylène, de la vapeur d'eau, de l'azote et de l'oxygène moléculaire, sur un catalyseur 2 d'oxydation du propylène. Le mélange 3 issu de cette réaction, comprenant les gaz n'ayant pas réagi, l'acroléine produite et des sous-produits, alimente un second réacteur comprenant un catalyseur 4 d'oxydation de l'acroléine en acide acrylique. Le second réacteur est éventuellement alimenté en en 9 par de l'oxygène ou de l'air. On obtient un mélange 10 comprenant l'acide acrylique produit, de l'acroléine résiduelle, les gaz n'ayant pas réagi, de l'eau et des sous-produits. Des fluides réfrigérants circulent en 6 et 7 de façon à maintenir une température de réaction pouvant être comprise entre 300°C et 380°C pour la réaction d'oxydation du propylène en acroléine et une température comprise entre 260°C et 320°C pour l'oxydation de l'acroléine en acide acrylique. Un échangeur thermique 8 permettant de refroidir les gaz de réaction issus de la première étape est placé dans le bas du premier réacteur. Un second échangeur thermique 8 est placé en aval des deux étapes d'oxydation. Les échangeurs 8 peuvent être à l'extérieur des réacteurs.

Conformément à un premier mode de réalisation du procédé selon l'invention, illustré de façon schématique sur la Figure 4, l'échangeur thermique 8 dans la configuration conventionnelle de la Figure 2, placé entre les deux lits catalytiques et permettant de refroidir le mélange gazeux issu de la réaction d'oxydation du propylène en acroléine, est remplacé par une étape de déshydratation de glycérol. Cette étape consiste à faire passer un mélange 11 constitué de glycérol sous forme de solution aqueuse vaporisée et éventuellement d'oxygène, en même temps que le mélange gazeux sortant de la zone d'oxydation comportant le catalyseur 2 d'oxydation du propylène en acroléine, sur un catalyseur 5 de déshydratation du glycérol. On obtient à la sortie de la zone comportant le catalyseur 5, un mélange d'acroléine résultant à la fois de la réaction d'oxydation du propylène et de la réaction de déshydratation du glycérol, ainsi que les sous-produits issus de ces deux réactions. Ce mélange passe ensuite sur le catalyseur 4 sur lequel l'acroléine est oxydée en acide acrylique. On obtient un mélange 10 comprenant l'acide acrylique produit, de l'acroléine résiduelle, les gaz n'ayant pas réagi, de l'eau et des sous-produits.

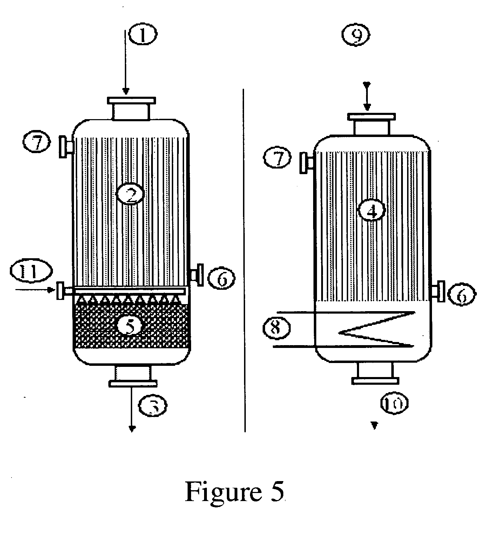

Conformément à un second mode de réalisation du procédé de l'invention, illustré de façon schématique sur la Figure 5, l'échangeur thermique 8 placé en aval du premier réacteur dans un procédé conventionnel d'oxydation du propylène en acide acrylique en deux étapes dans deux réacteurs consécutifs, tel que représenté sur la Figure 3, est remplacé par une étape de déshydratation de glycérol. Cette étape consiste à faire passer un mélange 11, constitué de glycérol sous forme de solution aqueuse vaporisée et éventuellement d'oxygène, en même temps que le mélange gazeux sortant de la zone d'oxydation comportant le catalyseur 2 d'oxydation du propylène en acroléine, sur un catalyseur 5 de déshydratation du glycérol. On obtient, à la sortie de la zone comportant le catalyseur 5, un mélange 3 d'acroléine résultant à la fois de la réaction d'oxydation du propylène et de la réaction de déshydratation du glycérol, ainsi que les sous-produits issus de ces deux réactions. Ce mélange 3 alimente le second réacteur comprenant le catalyseur 4 sur lequel l'acroléine est oxydée en acide acrylique. On obtient un mélange 10 comprenant l'acide acrylique produit, de l'acroléine résiduelle, les gaz n'ayant pas réagi, de l'eau et des sous-produits.

Selon le procédé de l'invention, il est possible d'obtenir un gain de productivité en acide acrylique de l'ordre de 50 à 200% par rapport aux procédés conventionnels.

On peut envisager de mettre en oeuvre une autre réaction endothermique que celle de déshydratation du glycérol pour récupérer de manière efficace la chaleur de réaction d'oxydation du propylène en acroléine. Notamment, la réaction d'oxydéshydratation de propane diol-1,3, ou la déshydratation de propanol-1 ou propanol-2, sont aussi intéressantes sous certains aspects, plus particulièrement si le lit de catalyseur de déshydratation est placé en amont du réacteur d'oxydation du propylène en acroléine. En effet, la déshydratation du propane diol-1,3 peut conduire à de l'alcool allylique qui, à son tour, peut s'oxyder sur le catalyseur d'oxydation du propylène en acroléine. Le propanol-1 ou le propanol-2, peut se déshydrater en propylène et s'oxyder ensuite en acroléine sur le catalyseur d'oxydation.

Les exemples suivants illustrent la présente invention sans toutefois en limiter la portée.

Dans les exemples, les produits formés, acroléine et acide acrylique sont analysés par chromatographie sur colonne capillaire EC-1000 montée sur un chromatographe HP6980 équipé d'un détecteur FID. L'analyse quantitative est effectuée avec un étalon externe.

### Exemple 1 :

On utilise une configuration de réacteur dans laquelle le glycérol est co-alimenté avec le mélange gazeux contenant le propylène du haut vers le bas, et comportant trois lits de catalyseur. Le réacteur en pyrex est muni d'un fritté pour retenir le catalyseur.

On charge tout d'abord une masse de 5 g de catalyseur d'oxydation d'acroléine en acide acrylique de référence ACS4 (de Nippon Shokubai), réduit en poudre dans une granulométrie de 100 à 160 microns, dilué avec 5 ml de carbure de silicium de granulométrie 0,125 mm. Ensuite on charge 9 ml de carbure de silicium de granulométrie 0,5 mm. Ensuite, on charge 6,498 g de catalyseur d'oxydation du propylène en acroléine de référence ACF4 (de Nippon Shokubai), dilué avec 7 ml de carbure de silicium de granulométrie 0,125 mm. Ensuite on charge différents lits de carbure de silicium de manière à séparer le catalyseur d'oxydation du propylène du catalyseur de déshydratation et contrôler indépendamment leur température : 2 ml de granulométrie 0,125 mm, puis 7 ml de granulométrie 0,5 mm et à nouveau 2 ml de 0,125 mm, et finalement 1 ml de 0,062 mm. Ensuite on charge 1,537 g de catalyseur de déshydratation de référence Z1044 (zircone tungstée de Dailchi Kigenso KK), dilué avec 4 ml de carbure de silicium de 0,062 mm. Et finalement, le réacteur est complété jusqu'en haut avec du carbure de silicium de granulométrie 0,125 mm (2ml) et 0,5 mm puis 1,19 mm.

Le réacteur est ensuite connecté à l'installation de test. Les températures des trois couches de catalyseur sont régulées de manière indépendante à 305 °C pour les deux couches supérieures de déshydratation du glycérol et d'oxydation du propylène, et à 280 °C pour la couche inférieure d'oxydation de l'acroléine en acide acrylique.

Le réacteur est alimenté par un mélange gazeux de propylène / oxygène / hélium-krypton / eau-glycérol avec des débits molaires horaires (exprimés en micromoles par heure) 30089 / 55584 / 288393 / eau : 53489 - glycérol : 4509. Le mélange gazeux hélium-krypton contient 4,92 % de krypton qui sert d'étalon interne. Le mélange eau-glycérol contient 30 % en poids de glycérol. Ces conditions représentent un débit molaire total de composés en C₃ (propylène + glycérol) de 34598 micromoles/h. Les effluents sont collectés en sortie du réacteur par un piège froid à glace et l'acroléine et l'acide acrylique produits sont dosés par analyse chromatographique.

Les effluents sont cumulés dans le piège pendant une durée de 80 minutes. Les gaz non condensables sont analysés pendant la durée du bilan. La quantité d'acroléine produite est de 503 micromolcs/h, et la quantité d'acide acrylique est de 26103 micromoles/h.

### Exemple 2 (Comparatif) :

On reproduit l'exemple 1, mais la solution aqueuse de glycérol est remplacée par de l'eau pure. Les débits molaires des réactifs sont alors : propylène / oxygène / hélium-krypton / eau-glycérol : 30089 / 55584 / 288393 / eau : 76666 - Glycérol : 0 (micromoles/h).

Les effluents sont cumulés dans le piège pendant une durée de 78 minutes. Les gaz non condensables sont analysés pendant la durée du bilan. La quantité d'acroléine produite est de 457 micromoles/h, et la quantité d'acide acrylique est de 23257 micromoles/h.

### Exemple 3 (Comparatif) :

On reproduit l'exemple 2, mais en remplaçant le catalyseur de déshydratation par du carbure de silicium. On utilise les mêmes conditions d'alimentation.

Les effluents sont cumulés dans le piège pendant une durée de 75 minutes. Les gaz non condensables sont analysés pendant la durée du bilan. La quantité d'acroléine produite est de 521 micromoles/h, et la quantité d'acide acrylique est de 23363 micromoles/h.

### Exemple 4 :

On utilise une configuration de réacteur comportant trois lits de catalyseur avec une alimentation du mélange gazeux contenant le propylène du haut vers le bas et une alimentation intermédiaire pour la solution de glycérol. Le réacteur en pyrex est muni d'un fritté pour retenir le catalyseur.

On charge tout d'abord une masse de 5 g de catalyseur d'oxydation d'acroléine en acide acrylique de référence ACS4 (de Nippon Shokubai), réduit en poudre dans une granulométrie de 100 à 160 microns, dilué avec 5 ml de carbure de silicium de granulométrie 0,125 mm. Ensuite on charge 9 ml de carbure de silicium de granulométrie 0,5 mm. Ensuite on charge une masse de 1,578 g de catalyseur de déshydratation du glycérol de référence Z1044 (zircone tungstée de DaiIchi Kigenso KK), dilué avec 4 ml de carbure de silicium de granulométrie 0,062 mm.

Ensuite on charge différents lits de carbure de silicium de manière à séparer le catalyseur de déshydratation du catalyseur d'oxydation du propylène et contrôler indépendamment leur température, et permettre l'injection d'une solution aqueuse de glycérol ou du glycérol hydraté entre les deux lits de catalyseur : 4 ml de granulométrie 0,125 mm, puis 7 ml de granulométrie 0,5 mm et à nouveau 2 ml de 0,125 mm. Ensuite on charge, 6,578 g de catalyseur d'oxydation du propylène en acroléine de référence ACF4 (de Nippon Shokubai), dilué avec 7 ml de carbure de silicium de granulométrie 0,125 mm. Et finalement, le réacteur est complété jusqu'en haut avec du carbure de silicium de granulométrie 0,125 mm (2 ml) et 0,5 mm puis 1,19 mm.

Le réacteur est ensuite connecté à l'installation de test. Les températures des couches de catalyseur sont régulées de manière indépendante à 260 °C pour les couches inférieures de déshydratation du glycérol et d'oxydation de l'acroléine en acide acrylique, et à 305 °C pour la couche supérieure d'oxydation du propylène en acroléine.

Le réacteur est alimenté par un mélange gazeux de propylène / oxygène / hélium-krypton / eau à sa partie supérieure avec des débits molaires horaires (exprimés en micromoles par heure) 30089 / 55584 / 288393 / 76666. Le mélange gazeux hélium-krypton contient 4,92 % de krypton qui sert d'étalon interne. Un mélange eau-glycérol contenant 80 % en poids de glycérol, est alimenté entre la couche de catalyseur d'oxydation du propylène et b catalyseur de déshydratation. avec un débit glycérol / eau :4530 / 5794 (micromoles/h). Ces conditions représentent un débit molaire total de composés en C₃ (propylène + glycérol) de 34619 micromoles/h.

Les effluents sont collectés en sortie du réacteur par un piège froid à glace et l'acroléine et l'acide acrylique produits sont dosés par analyse chromatographique.

Les effluents sont cumulés dans le piège pendant une durée de 84 minutes. Les gaz non condensables sont analysés pendant la durée du bilan. La quantité d'acroléine produite est de 389 micromoles/h, et la quantité d'acide acrylique est de 26360 micromoles/h. Le propylène résiduel est de 2613 micromoles/h.

### Exemple 5 :

On reproduit l'exemple 4, mais en utilisant une solution de glycérol à 95 % poids (glycérol hydraté).

Les débits molaires horaires (en micromoles par heure) des constituants du mélange sont les suivants : propylène / oxygène / hélium-krypton / eau : 30089 / 55584 / 288393 / 76666 pour l'alimentation supérieure, et glycérol / eau : 8220 / 2205 pour l'alimentation intermédiaire. Ces conditions représentent un débit molaire total de composés en C₃ (propylène + glycérol) de 38309 micromoles/h.

Les effluents sont cumulés dans le piège pendant une durée de 81 minutes. Les gaz non condensables sont analysés pendant la durée du bilan. La quantité d'acroléine produite est de 633 micromoles/h, et la quantité d'acide acrylique est de 29898 micromoles/h. Le propylène résiduel est de 2803 micromoles/h.

### Exemple 6 :

On reproduit l'exemple 4, mais en utilisant une solution de glycérol à 70 % en poids. Les débits molaires horaires (en micromoles par heure) des constituants du mélange sont les suivants : propylène / oxygène / hélium-krypton / eau : 30089 / 55584 / 288393 / 76666 pour l'alimentation supérieure et glycérol / eau : 6350 / 13923. Ces conditions représentent un débit molaire total de composés en C₃ (propylène + glycérol) de 36439 micromoles/h.

Les effluents sont cumulés dans le piège pendant une durée de 78 minutes. Les gaz non condensables sont analysés pendant la durée du bilan. La quantité d'acroléine produite est de 612 micromoles/h, et la quantité d'acide acrylique est de 28212 micromoles/h. Le propylène résiduel est de 2702 micromoles/h.

## Revendications

1. Procédé de préparation d'acide acrylique à partir du propylène comprenant une première étape d'oxydation du propylène en acroléine et une seconde étape d'oxydation de l'acroléine en acide acrylique, **caractérisé en ce qu'**il comprend une étape de déshydratation de glycérol en présence d'un gaz contenant du propylène.

2. Procédé selon la revendication 1, **caractérisé en ce que** le gaz contenant du propylène est le gaz de réaction issu de l'étape d'oxydation du propylène en acroléine.

3. Procédé selon la revendication 1 ou 2, **caractérisé en ce que** la réaction de déshydratation est effectuée en phase gaz en présence d'un catalyseur.

4. Procédé selon l'une quelconque des revendications précédentes, **caractérisé en ce que** l'on ajoute de l'oxygène moléculaire pour l'étape de déshydratation du glycérol.

5. Procédé selon l'une quelconque des revendications précédentes, **caractérisé en ce que** le glycérol est injecté sous forme liquide ou sous forme gazeuse.

6. Procédé selon l'une quelconque des revendications précédentes, **caractérisé en ce que** lon utilise du glycérol pur, ou sous forme de solution aqueuse concentrée ou diluée.

7. Procédé selon l'une quelconque des revendications précédentes, **caractérisé en ce que** l'oxydation du propylène est réalisée en présence d'un ballast thermique.

## Claims

1. A process for the preparation of acrylic acid from propylene comprising a first stage of oxidation of the propylene to give acrolein and a second stage of oxidation of the acrolein to give acrylic acid, **characterized in that** it comprises a stage of dehydration of glycerol in the presence of a gas comprising propylene.

2. The process as claimed in claim 1, **characterized in that** the gas comprising propylene is the reaction gas resulting from the stage of oxidation of the propylene to give acrolein.

3. The process as claimed in claim 1 or 2, **characterized in that** the dehydration reaction is carried out in the gas phase in the presence of a catalyst.

4. The process as claimed in any one of the preceding claims, **characterized in that** molecular oxygen is added for the stage of dehydration of the glycerol.

5. The process as claimed in any one of the preceding claims, **characterized in that** the glycerol is injected in the liquid form or in the gas form.

6. The process as claimed in any one of the preceding claims, **characterized in that** use is made of pure glycerol or glycerol in the form of a concentrated or dilute aqueous solution.

7. The process as claimed in any one of the preceding claims, **characterized in that** the oxidation of the propylene is carried out in the presence of a thermal ballast.

## Patentansprüche

1. Verfahren zur Herstellung von Acrylsäure ausgehend von Propylen, das eine erste Stufe der Oxidation des Propylens zu Acrolein und eine zweite Stufe der Oxidation des Acroleins zu Acrylsäure umfasst,
**dadurch gekennzeichnet, dass** es eine Stufe der Wasserabspaltung aus Glycerin in Gegenwart eines Propylen enthaltenden Gases umfasst.

2. Verfahren nach Anspruch 1, **dadurch gekennzeichnet, dass** das Propylen enthaltende Gas das aus der Stufe der Oxidation des Propylens zu Acrolein resultierende Reaktionsgas ist.

3. Verfahren nach Anspruch 1 oder 2, **dadurch gekennzeichnet, dass** die Reaktion der Wasserabspaltung in der Gasphase in Gegenwart eines Katalysators vorgenommen wird.

4. Verfahren nach einem der vorhergehenden Ansprüche, **dadurch gekennzeichnet, dass** bei der Stufe der Wasserabspaltung aus Glycerin molekularer Sauerstoff zugegeben wird.

5. Verfahren nach einem der vorhergehenden Ansprüche, **dadurch gekennzeichnet, dass** das Glycerin in flüssiger Form oder als Gas eingeführt wird.

6. Verfahren nach einem der vorhergehenden Ansprüche, **dadurch gekennzeichnet, dass** das Glycerin in reiner Form oder in Form einer konzentrierten oder verdünnten wässerigen Lösung eingesetzt wird.

7. Verfahren nach einem der vorhergehenden Ansprüche, **dadurch gekennzeichnet, dass** die Oxidation des Propylens in Gegenwart eines thermischen Ballasts durchgeführt wird.
